# FASCICULE DE BREVET EUROPEEN

(11) **EP 0 762 865 B1**
(45) Date de publication et mention de la délivrance du brevet: **05.08.1998**
(21) Numéro de dépôt: 95921874.4
(22) Date de dépôt: 01.06.1995
(51) Int. Cl.: A61J 3/06, B01J 2/04

(54) **PROCEDE DE PREPARATION ET PERLES OBTENUES CONTENANT UN PRINCIPE ACTIF PRESENTANT UN POINT DE FUSION NON DEFINI**
VERFAHREN ZUR HERSTELLUNG VON PERLEN WELCHE EINEN AKTIVEN BESTANDTEIL AUFWEISEN MIT EINEM NICHT DEFINIERTEN SCHMELZPUNKT
METHOD FOR THE PREPARATION OF PEARLS AND PEARLS OBTAINED CONTAINING AN ACTIVE INGREDIENT WITH AN UNDEFINED MELTING POINT

(30) Priorité: 03.06.1994 FR 9406842
(43) Date de publication de la demande: 19.03.1997
(73) Titulaire: RHONE-POULENC RORER S.A., 92160 Antony (FR)
(72) Inventeur: DELEUIL, Michel, F-92160 Antony (FR); LE THIESSE, Jean-Claude, F-42100 Saint-Etienne (FR)
(86) Numéro de dépôt international: FR9500717
(87) Numéro de publication internationale: WO9533433

(56) Documents cités:
- BE-A- 628 183
- US-A- 5 188 838

## Description

La présente invention concerne un nouveau procédé de préparation de perles d'un composé présentant un point de cristallisation non défini présentant un profil de libération rapide et/ou prolongée. Elle concerne également les perles obtenues par le procédé.

Il est connu selon le brevet US 5 188 838 de préparer de perles d'un dérivé d'un acide arylpropionique présentant un point de cristallisation non défini, notamment le kétoprofène, par un procédé qui consiste à mélanger sous forme fondue le principe actif avec un excipient pharmaceutique, à forcer le passage de la masse fondue à travers une buse qui subit une vibration, à laisser tomber dans une tour, à contrecourant avec un gaz, les perles formées puis à rassembler les perles formées dans le bas de la tour.

On entend par composé présentant un point de cristallisation non défini un phénomène connu sous le nom de surfusion, le point de fusion du composé est parfaitement défini mais son point de cristallisation ne l'est pas. En effet la cristallisation, par exemple du kétoprofène, en tant que produit chimique pur demande plusieurs jours, surtout si la masse fondue est placée dans un réfrigérateur. Or lors du passage d'un mélange contenant le kétoprofène et un ou plusieurs excipients dans la tour, les gouttes formées se solidifient suffisamment avant d'atteindre le bas de la tour, ce qui permet de préparer le kétoprofène sous une forme sphérique solide. En réalité la perle présente un aspect extérieur solide qui permet sa manipulation de façon facile, mais le kétoprofène se trouve à l'intérieur sous une forme non cristallisée. La cristallisation demande alors pour s'effectuer totalement sans intervention de l'homme encore des semaines. Dans le brevet précédemment cité est adjoint éventuellement en bas de la tour un lit fluidisé qui permet une cristallisation plus rapide du kétoprofène.

Il est apparu de façon tout à fait étonnante que la nature chimique de l'excipient, le rapport pondéral entre le kétoprofène et l'excipient, et surtout la dimension de la perle formée et la température de fluidisation des perles obtenues avaient un effet important sur la vitesse de libération du kétoprofène à partir de la perle obtenue.

La présente invention a permis de préparer des perles de principes actifs présentant un phénomène de surfusion (point de cristallisation non défini) qui ont un profil de libération reproductible.

Parmi les principes actifs présentant un point de cristallisation non défini on peut citer à titre uniquement illustratif : le kétoprofène, l'ibuprofène, l'acétyl para aminophénol.

L'excipient utilisé dans le cadre de la présente invention est choisi parmi les excipients décrits dans le brevet US 5 188 838 et plus préférentiellement parmi les acides gras saturés contenant 12 à 18 atomes de carbone. On préfère tout particulièrement utiliser l'acide stéarique.

Ces perles sont préparées selon un procédé tel que décrit dans le brevet US 5 188 838, le lit fluidisé qui est adjoint éventuellement à la sortie de la tour est utilisé dans des conditions de température strictes. Pour obtenir des perles à libération prolongée la température du lit est maintenue en dessous de la température de début de liquifaction de l'excipient et au dessus de la température de transition vitreuse du principe actif pour permettre sa cristallisation. Pour obtenir des perles à libération rapide la température du lit est maintenue quelques degrés au dessus de la température de début de liquéfaction de l'excipient.

Un des premiers objets de l'invention consiste donc en un procédé de préparation de perles d'un principe actif présentant un point de fusion non défini, perles présentant un profil de libération prolongé dans lequel on introduit dans les récipients d'alimentation d'une tour de "prilling" le principe actif présentant un point de fusion non défini et un excipient de cristallisation, on passe le mélange à travers une buse vibrée pour former des perles parfaitement calibrées que l'on laisse tomber dans la tour à contre courant avec de l'air froid caractérisé en ce que l'on adjoint en bas de la tour un lit fluidisé dont la température est maintenue en dessus de la température de transition vitreuse du principe actif présentant un point de fusion non défini et en dessous de la température du début de liquéfaction de l'excipient.

Un deuxième objet de l'invention consiste à préparer des perles d'un principe actif présentant un point de fusion non défini, perles présentant un profil de libération rapide dans lequel on introduit dans les récipients d'alimentation d'une tour de "prilling" le principe actif présentant un point de fusion non défini et un excipient de cristallisation, on passe le mélange à travers une buse vibrée peur former les perles que l'on laisse tomber dans la tour à contre courant avec de l'air froid caractérisé en ce que l'on adjoint en bas de la tour un lit fluidisé dont la température est maintenue au dessus de la température du début de liquéfaction de l'excipient.

On entend par température de transition vitreuse le phénomène physique se traduisant notamment par une brusque variation de la capacité calorifique du produit. La température à laquelle se produit cette transition peut être déterminée par analyse thermique différentielle. Le produit solide présentant un phénomène de surfusion est fondu puis amené en état de surfusion à une température très basse (par exemple -50°C). Quand on remonte progressivement la température, il apparaît un brusque décalage de la ligne de base qui correspond à la variation de la capacité calorifique du produit. La température à laquelle la ligne de base évolue est appelée température de transition vitreuse.

On entend par température de début de liquéfaction de l'excipient la température à laquelle le mélange excipient-principe actif, d'abord fondu puis refroidi rapidement à une température inférieure à la température de fusion de l'excipient (20°C minimum) présente un début de changement d'état physique (soit dissolution de l'excipient dans le principe actif resté à l'état de surfusion, soit fusion de l'excipient) quand on élève de nouveau la température de l'échantillon. Cette détermination est réalisée par analyse thermique différentielle du mélange.

Il est bien évident que la température de début de liquéfaction de l'excipient dépend de la concentration en principe actif non cristallisé dans le mélange. Ainsi, au fur et à mesure de la cristallisation du principe actif pendant la phase de fluidisation des perles, la température de début de liquéfaction de l'excipient évolue vers des températures plus élevées s'approchant de la température de fusion de l'excipient pur.

Pour une meilleure mise en oeuvre de l'invention, on introduit le principe actif présentant un point de fusion non défini et l'excipient dans un des récipients d'alimentation de la tour, les récipients d'alimentation de la tour sont maintenus sous une atmosphère de gaz inerte. A l'état fondu les deux produits sont notamment miscibles et donnent un mélange homogène. Au moyen de deux tubes les liquides fondus sont amenés au dessus d'une buse qui est maintenue dans une atmosphère non refroidie et qui est même éventuellement chauffée. La buse présente 1 à 50 perforations ou plus présentant un' diamètre compris entre 20 et 5000 microns et de préférence compris entre 250 et 800 microns. La longueur de la perforation est de préférence comprise entre 0,5 et 10 fois le diamètre de celle-ci.

Cette buse est soumise à un système de vibration électrique de haute féquence (50 à 10000 hertz). L'air froid qui permet la solidification du principe actif présentant un point de fusion non défini et de l'excipient est introduit en bas de tour et sort en-dessous la buse à une distance d'environ L/10 par rapport au sommet de la tour, L étant la hauteur de la tour. La hauteur de la tour varie entre 1 mètre et une dixaine de mètres, la tour peut comporter au quart inférieur de sa hauteur une jupe perforée tronconique qui centre les perles dans le lit fluidisé.

La tour possède en bas un lit fluide, ce lit a de préférence une forme tronconique équipé à la base d'une grille de distribution permettant de minimiser l'adhérence sur les parois et de favoriser les chocs paroi perles dans le but d'accroître la vitesse de solidification. L'adjonction de ce type d'appareil permet une solidification plus rapide du mélange entre le principe actif présentant un point de fusion non défini et l'excipient dans la bille, la température du lit est réglée en fonction de la nature du profil de libération désiré.

Il est apparu de façon tout à fait étonnante que bien que la solidification de la bille soit favorisée par l'utilisation d'un lit fluidisé, selon la température d'utilisation de ce lit les perles obtenues avaient un comportement totalement différent.

Ainsi, si la température du lit ou celle du stockage permet une liquéfaction au moins partielle de l'excipient, des échanges solide/liquide peuvent avoir lieu et on assiste à une totale redistribution des phases principe actif/excipient. La croissance cristalline peut se développer et la cristallisation du principe actif conduit à le formation d'une structure en forme de géode. Le volume poreux de ces perles est généralement supérieur à 0, 045 µl/g. Ces perles présentent un profil de libération rapide.

Par contre, si la température du lit est inférieure au début de la température de liquéfaction de l'excipient, le principe actif en surfusion qui présente une viscosité élevée reste piégé dans la matrice d'excipient solidifé, la croissance cristalline ne peut se développer et le principe actif reste finement dispersé au sein de l'excipient. Sa libération ne se fera que de façon prolongée. La perle a une structure interne nuclée.

Ces perles ont une libération encore plus ralentie lorsqu'elles sont constituées de kétoprofène et d'acide stéarique ayant un point de fusion supérieur à 55°C et qu'elles présentent une granulométrie supérieure à 0,7 mm de préférence supérieure à 1 mm et encore plus préférentiellement supérieure à 1,5mm, c'est dire quand la buse présente des perforations ayant un diamètre supérieur à 0,4 mm et de préférence supérieur à 0,8 mm. La libération est aussi ralentie, pour les mêmes conditions de fluidisation, quand l'excipient présente un point de fusion plus élevé et quand le rapport principe actif sur excipient diminue et de préférence est inférieur à 1. La libération est ralentie quand le volume poreux de ces perles est inférieur à 0,040 µl/g.

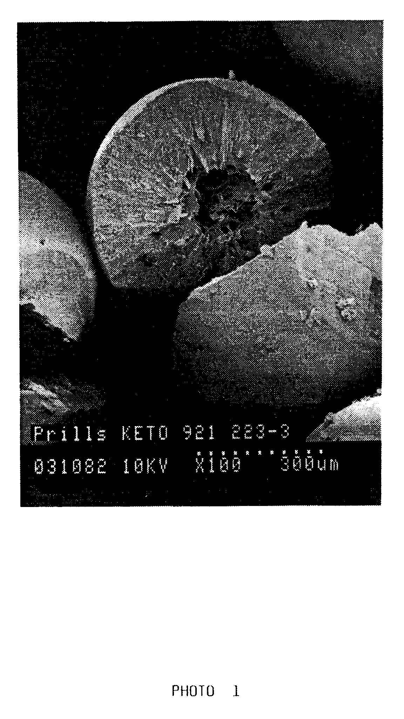

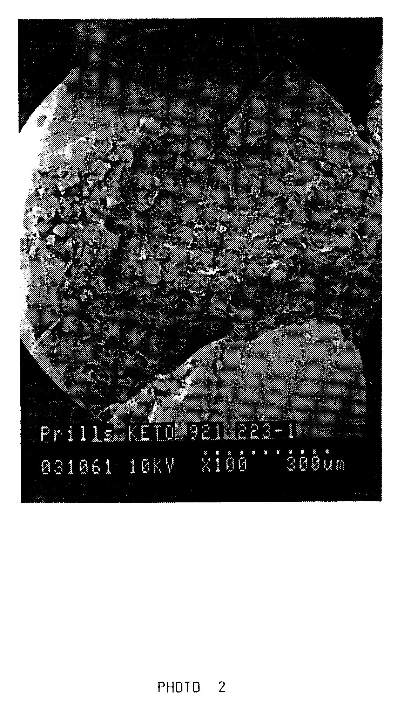

La structure de ces deux sortes de perles est clairement identifiée sur les photographies annexées. La photo 1 représente une perle à libération rapide, la photo 2 une perle à libération prolongée.

Les perles présentant une structure nuclée et qui ont un profil de libération prolongé peuvent être modifiées de façon à présenter une double cinétique de libération. Dans ce cas les perles à libération prolongée sont enrobées par un mélange contenant un polymère hydrophile et le principe actif. Ce polymère hydrophile peut être choisi parmi l'hydroxypropylméthylcelllulose ou la polyvinylpyrrolidone, il est notamment appliqué sous forme d'une suspension aqueuse d'un mélange de principe actif pulvérulent et dudit polymère. On préfère utiliser l'hydroxypropylméthylcellulose.

On préfére utiliser une suspension aqueuse contenant en poids :
- principe actif 70-98 %
- hydroxypropylméthylcelllulose 2-30 %

La suspension est préparée dans un réacteur agité par exemple du type agitateur Heidolph puis dans une turbine par exemple de type Ultra-Turrax. On peut avantageusement ajouter à la suspension aqueuse de principe actif un plastifiant hydrophile tel que par exemple le polyéthylène glycol et un désintégrant tel que la carboxyméthylcellulose. Ces additifs éventuels sont ajoutés selon des quantités pondérales notamment comprises entre 1 et 2 % par rapport aux autres composés solides de la suspension.

La suspension est pulvérisée sur les perles dans un lit fluidisé par exemple de type Wurster.

La présente invention sera plus complètement décrite à l'aide des exemples suivants qui ne doivent pas être considérés comme limitatifs de l'invention.

### EXEMPLES

Dans une tour de "prilling" muni d'un fondoir, on introduit des mélanges kétoprofène/excipient dont la nature est indiquée dans le tableau 1. Ces mélanges sont introduits dans le recipient, fondus et maintenus sous atmosphère d'azote. Au moyen de tubes, les liquides fondus sont amenés au-dessus d'une buse qui est maintenue dans une atmosphère non refroidie et qui est même éventuellement chauffée. La buse utilisée présente une seule perforation présentant un diamètre compris entre 250 et 550 µm. La longueur de la perforation est comprise entre 0,5 et 10 fois le diamètre de celle-ci. Cette buse est soumise à un système de vibration électrique de haute fréquence (50 à 10000 hertz). L'air froid est introduit en bas de la tour et sort en-dessous de la buse à une distance de préférence égale à environ L/10 par rapport au sommet de la tour, L étant la hauteur de la tour. La hauteur de la tour varie entre 1 mètre et une dizaine de mètres.

Le lit fluidisé qui est ajouté en bas de la tour est de préférence un lit fluidisé en forme d'entonnoir équipé à sa base d'une grille de distribution permettant de minimiser l'adhérence sur les parois et de favoriser les chocs paroi-perles dans le but d'accroître la vitesse de solidification. Le tableau 1 indique les vitesses de libération du kétoprofène en fonction de la température de fluidisation, de la vitesse de montée en température et du titre en acide stéarique de l'excipient utilisé (ici l'acide stéarique présente un titre de 92 %). Le tableau 2 indique la vitesse de libération du kétoprofène en fonction du diamètre de la buse et du titre en acide stéarique de l'excipient utilisé.

L'exemple ci-après démontre l'influence de la cinétique de montée en température :
- mélange 60/40 kétoprofène/acide stéarique
- buse de 400 µm de diamètre
- température d'air dans la tour : -5°C
- réception des perles en bas de la tour dans un lit fluidisé à -10°C.

Protocole de fluidisation 1 : Les perles en fluidisation à -10°C en bas de tour sont ramenées à température ambiante an une dizaine de minutes et fluidisées à température ambiante (environ 18°C) pendant 30 minutes. La température est ensuite élevée lentement jusqu'à 45°C (en 2 heures environ) et les perles sont maintenues en fluidisation à cette température pendant 4 heures. Le temps de dissolution de 80 % du kétoprofène est de 3,9 heures.
Protocole de fluidisation 2 : Les perles sont fluidisées 4 heures à 45°C selon le protocole 1. La température est ensuite élevée en une heure environ jusqu'à 55°C et les perles sont maintenues en fluidisation à cette température pendant 2 heures. Le temps de dissolution de 80 % du kétoprofène est de 3,1 heures.
Protocole de fluidisation 3 : Les perles en fluidisation à -10°C en bas de tour sont tranferées brusquement dans un second lit fluidisé préchauffé à 55°C. Les perles sont maintenues en fluidisation à cette température pendant 2 heures. Le temps de dissolution de 80% du kétoprofène est de 1,9 heures.
Les perles des exemples 1 à 6 du tableau 1 sont préparées selon un protocole voisin du protocole de fluidisation 1. Les conditions de fluidisation et le temps de libératon de 80% du kétoprofène sont indiqués dans le tableau 1.
Les perles obtenues aux exemples 1 à 5 sont enrobées par les mélanges indiqués dans le tableau 4. Le rapport pondéral entre le kétoprofène contenu dans la couche rapide et dans la perle à libération prolongée est de 25/75. Les conditions opératoires appliquées lors de cet enrobage, la quantité de suspension pulvérisée et la vitesse de libération du kétoprofène à partir de la couche rapide sont indiqués dans le tableau 4.

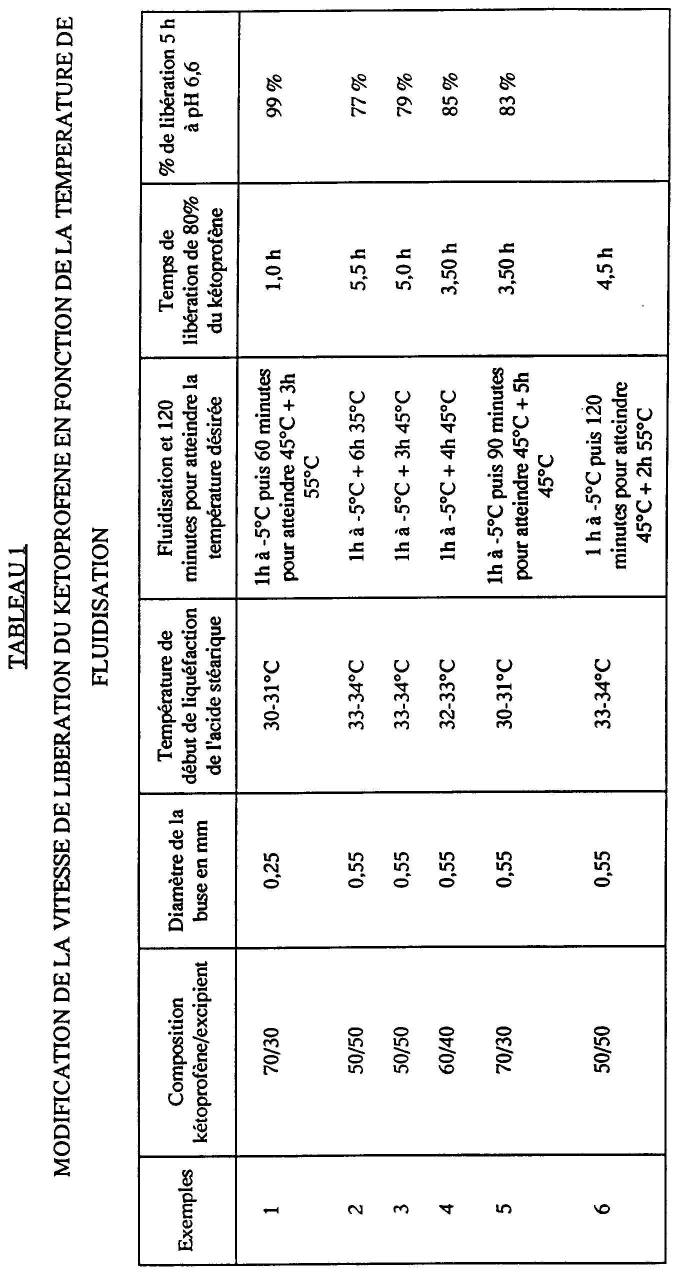

**TABLEAU 2**

| VARIATION DE LA VITESSE DE LIBERATION DU KETOPROFENE EN FONCTION DU DIAMETRE DE LA BUSE | | | | | |
|---|---|---|---|---|---|
| Exemples | Composition kétoprofène/ac. stéarique | Conditions de fluidisation | Diamètre de la buse en mm | | |
| | | | 0,25 | 0,40 | 0,55 |
| 7, 8, 9 | Acide stéarique 55 % 50/50 | 2h-5°C + 2h 45°C | 1,2 h | 2,6 h | 3,3 h |
| 10, 11, 12 | Acide stéarique 98 % 50/50 | 2h-5°C + 2h 45°C | 1,6 h | 5,1 h | 7,1 h |
| 13, 14, 15 | Acide stéarique 55 % 70/30 | 2h-5°C + 2h 45°C | 1,3 h | 1,8 h | 2,6 h |
| 16 | Acide stéarique 98 % 70/30 | 2h-5°C + 2h 45°C | | 2,9 h | |

**TABLEAU 3**

| VARIATION DE LA VITESSE DE LIBERATION DU KETOPROFENE AVEC LE VOLUME POREUX | | | | | |
|---|---|---|---|---|---|
| Exemples | Composition kétoprofène/excipient acide stéarique à 55 % | Fluidisation | Stockage | Volume poreux | Temps de libération |
| 17 | 70/30 | 2h 20°C | 20°C | 0,038 | 8,2 h |
| 18 | 70/30 | 1h 0°C | 20°C | 0,035 | 7,2 h |
| 19 | 70/30 | 2h 20°C | 45°C | >0,050 | 2,5 h |
| 20 | 70/30 | 1h 0°C | 45°C | 0,049 | 1,9 h |
| 21 | 50/50 | 2h 20°C | 20°C | 0,031 | 3,1 h |
| 22 | 50/50 | 2h 20°C | 45°C | 0,044 | 1,7 h |

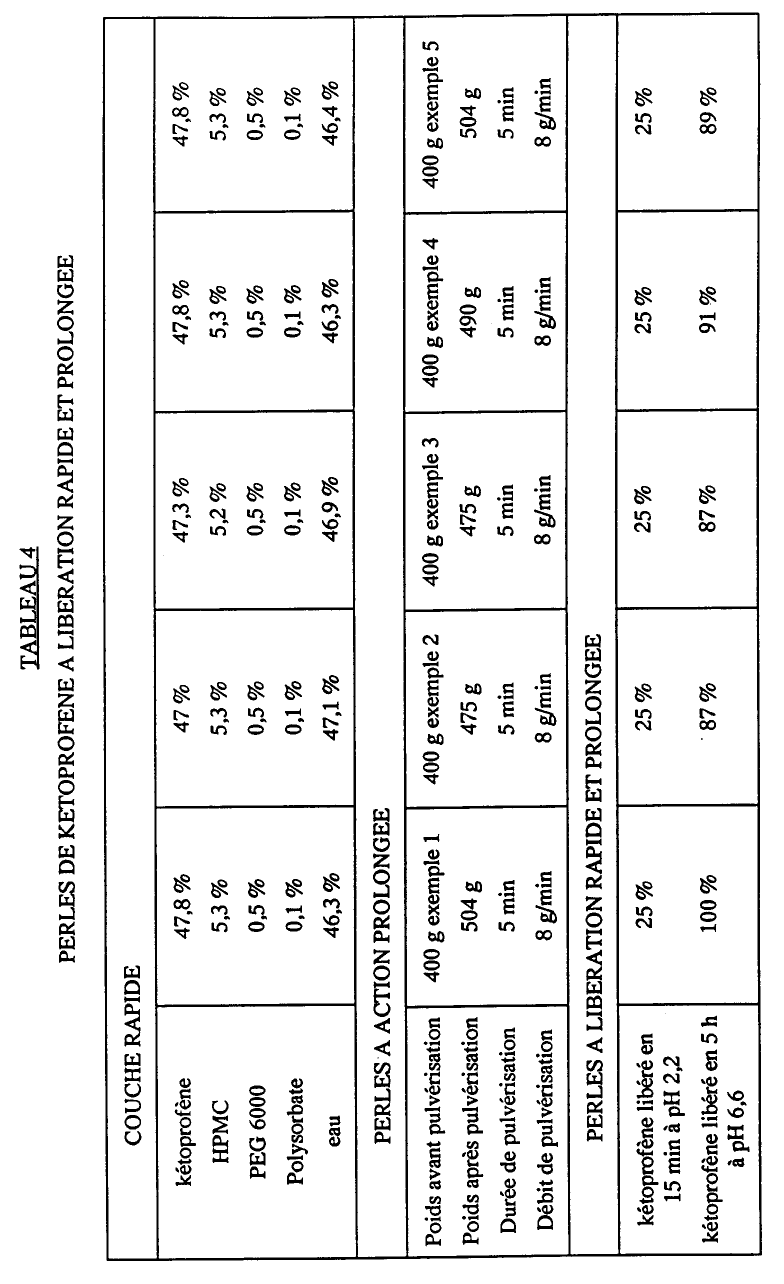

## Revendications

1. Procédé de préparation de perles d'un principe actif présentant un point de fusion non défini, perles présentant un profil de libération prolongé dans lequel on introduit dans les récipients d'alimentation d'une tour de "prilling" le principe actif présentant un point de fusion non défini et un excipient de cristallisation, on passe le mélange à travers une buse vibrée pour former les perles que l'on laisse tomber dans la tour à contre courant avec de l'air froid et l'on adjoint en bas de la tour un lit fluidisé, caractérisé en ce que la température dudit lit fluidisé est maintenue au-dessus de la température de transition vitreuse du principe actif présentant un point de fusion non défini et en dessous de la température du début de liquéfaction de l'excipient.

2. Procédé de préparation de perles d'un principe actif présentant un point de fusion non défini, perles présentant un profil de libération rapide dans lequel on introduit dans les récipients d'alimentation d'une tour de "prilling" le principe actif présentant un point de fusion non défini et un excipient de cristallisation, on passe le mélange à travers une buse vibrée pour former les perles que l'on laisse tomber dans la tour à contre courant avec de l'air froid et l'on adjoint en bas de la tour un lit fluidisé, caractérisé en ce que la température dudit lit fluidisé est maintenue au-dessus de la température du début de liquéfaction de l'excipient.

3. Procédé de préparation selon la revendication 1 caractérisé en ce que la buse présente un diamètre supérieur à 0,4 mm.

4. Perles de kétoprofène à libération prolongée obtenues selon l'une quelconque des revendications 1 et 3 caractérisées en ce qu'elles sont constituées de kétoprofène et d'acide stéarique ayant un point de fusion supérieur à 55°C et qu'elles ont un diamètre au moins égal à 700 µm et de préférence au moins égal à 1000 µm.

5. Perles de kétoprofène à libération prolongée obtenues selon l'une quelconque des revendications 1 et 3 caractérisées en ce qu'elles ont un volume poreux inférieur à 0,040 µl/g.

6. Perles de principe actif présentant un point de cristallisation non défini qui présentent une libération prolongée et une libération immédiate caractérisées en ce qu'elles sont constituées de perles selon l'une quelconque des revendications 1 et 3 à 5 enrobées par une suspension aqueuse contenant le principe actif et un polymère hydrophile.

7. Perles selon la revendication 6 caractérisées en ce que le polymère hydrophile est choisi parmi l'hydroxypropylméthylcellulose.

## Claims

1. Process for the preparation of prills of an active principle exhibiting an indefinite melting point, prills exhibiting a prolonged release profile, in which the active principle exhibiting an indefinite melting point and a crystallisation excipient are introduced into the feed receptacles of a prilling tower and the mixture is passed through a nozzle which is vibrated to form the prills which are allowed to fall in the tower counter-currentwise to cold air and added at the bottom of the tower there is a fluidized bed, characterized in that the temperature of the said fluidized bed is maintained above the glass transition temperature of the active principle exhibiting an indefinite melting point and below the temperature of onset of liquefaction of the excipient.

2. Process for the preparation of prills of an active principle exhibiting an indefinite melting point, prills exhibiting a rapid release profile, in which the active principle exhibiting an indefinite melting point and a crystallization excipient are introduced into the feed receptacles of a prilling tower and the mixture is passed through a nozzle which is vibrated to form the prills which are allowed to fall in the tower counter-currentwise to cold air and added at the bottom of the tower there is a fluidized bed, characterized in that the temperature of the said fluidized bed is maintained above the temperature of onset of liquefaction of the excipient.

3. Process of preparation according to Claim 1, characterized in that the nozzle has a diameter larger than 0.4 mm.

4. Prills of ketoprofen with prolonged release which are obtained according to either of Claims 1 and 3, characterized in that they consist of ketoprofen and of stearic acid with a melting point higher than 55°C and in that they have a diameter at least equal to 700 µm and preferably at least equal to 1000 µm.

5. Prills of ketoprofen with prolonged release which are obtained according to either of Claims 1 and 3, characterised in that they have a pore volume lower than 0.040 µl/g.

6. Prills of active principle exhibiting an indefinite crystallization point, which exhibit a prolonged release and an immediate release, characterised in that they consist of prills according to any one of Claims 1 and 3 to 5, coated with an aqueous suspension containing the active principle and a hydrophilic polymer.

7. Prills according to Claim 6, characterised in that the hydrophilic polymer is chosen from hydroxypropyl methyl cellulose.

## Patentansprüche

1. Verfahren zur Herstellung von Perlen aus einem aktiven Bestandteil, der einen nicht definierten Schmelzpunkt aufweist, Perlen, die ein verlängertes Freisetzungsprofil aufweisen, bei dem man in die Zuführungsbehälter eines "Prill"-Turms den aktiven Bestandteil, der einen nicht definierten Schmelzpunkt aufweist, und einen Kristallisationsexzipienten einführt, man das Gemisch durch eine Vibrationsdüse befördert, um die Perlen zu bilden, die man im Gegenstrom mit kalter Luft in den Turm fallen läßt, und man am Fuß des Turms ein Fließbett zufügt, dadurch gekennzeichnet, daß die Temperatur des Fließbetts oberhalb der Glasübergangstemperatur des aktiven Bestandteils, der einen nicht definierten Schmelzpunkt aufweist, und unterhalb der Temperatur des Verflüssigungsbeginns des Exzipienten gehalten wird.

2. Verfahren zur Herstellung von Perlen aus einem aktiven Bestandteil, der einen nicht definierten Schmelzpunkt aufweist, Perlen, die ein schnelles Freisetzungsprofil aufweisen, bei dem man in die Zuführungsbehälter eines "Prill"-Turms den aktiven Bestandteil, der einen nicht definierten Schmelzpunkt aufweist, und einen Kristallisationsexzipienten einführt, man das Gemisch durch eine Vibrationsdüse befördert, um die Perlen zu bilden, die man im Gegenstrom mit kalter Luft in den Turm fallen läßt, und man am Fuß des Turms ein Fließbett zufügt, dadurch gekennzeichnet, daß die Temperatur des Fließbetts oberhalb der Temperatur des Verflüssigungsbeginns des Exzipienten gehalten wird.

3. Verfahren zur Herstellung gemäß Anspruch 1, dadurch gekennzeichnet, daß die Düse einen Durchmesser größer als 0,4 mm aufweist.

4. Perlen aus Ketoprofen mit verlängerter Freisetzung, die gemäß einem der Ansprüche 1 und 3 erhalten werden, dadurch gekennzeichnet, daß sie aus Ketoprofen und Stearinsäure mit einen Schmelzpunkt höher als 55 °C bestehen und daß sie einen Durchmesser von wenigstens gleich 700 µm und vorzugsweise von wenigstens gleich 1000 µm haben.

5. Perlen aus Ketoprofen mit verlängerter Freisetzung, die gemäß einem der Ansprüche 1 und 3 erhalten werden, dadurch gekennzeichnet, daß sie ein Porenvolumen kleiner als 0,040 µl/g haben.

6. Perlen aus aktivem Bestandteil, der einen nicht definierten Kristallisationspunkt aufweist, die eine verlängerte Freisetzung und eine sofortige Freisetzung aufweisen, dadurch gekennzeichnet, daß sie aus Perlen gemäß einem der Ansprüche 1 und 3 bis 5 bestehen, die mit einer wäßrigen Suspension, die den aktiven Bestandteil und ein hydrophiles Polymer enthält, umhüllt sind.

7. Perlen gemäß Anspruch 6, dadurch gekennzeichnet daß das hydrophile Polymer unter Hydroxypropylmethylcellulose ausgewählt ist.
